# EUROPEAN PATENT SPECIFICATION

(11) **EP 3 030 295 B1**
(45) Date of publication and mention of the grant of the patent: **31.05.2017**
(21) Application number: 13783105.3
(22) Date of filing: 08.08.2013
(51) Int. Cl.: A61M 11/06, A61M 11/00, B05B 1/16, B05B 1/26

(54) **NEBULIZER VIAL FOR AEROSOL THERAPY**
VERNEBLERPHIOLE FÜR DIE AEROSOLTHERAPIE
FLACON DE NÉBULISEUR POUR THÉRAPIE PAR AÉROSOLS

(43) Date of publication of application: 15.06.2016
(73) Proprietor: MED 2000 S.r.l., 25080 Padenghe Sul Garda BS (IT)
(72) Inventor: FRACCAROLI Davide, I-25010 Desenzano del Garda (BS) (IT)
(74) Representative: Chimini, Francesco
(86) International application number: PCT/IT2013/000223
(87) International publication number: WO 2015/019371

(56) References cited:
- EP-A2- 0 261 649
- WO-A1-02/13895

## Description

The present invention relates to a nebulizer vial for aerosol therapy.

As is known, nebulizer vials for aerosol therapy use the Venturi principle, in particular, the so-called double Venturi. The air coming from a compressor is made to pass at high speed through a tube of small diameter which narrows internally (to form the Venturi) and ends with an orifice which emerges from the free surface of a solution of a medication for aerosol therapy contained in a reservoir. A nozzle (also called "pisper" in the technical jargon) is concentrically fitted onto the end part of the aforesaid tube so that an interspace remains between the two which communicates at the bottom with the solution contained in the reservoir. The compressed air coming out of the orifice of said tube sucks the particles or droplets of the medication solution upwards through the aforementioned interspace, forming a flow of particles of solution directed upwards, which comes out of the orifice of the nozzle.

The atomisation (to form the aerosol) of the flow of particles of solution thus formed is then obtained by making such flow strike against a flow-breaker placed above the orifice. Usually, the flow breaker device is positioned at the lower end of a duct, without closing it entirely however, external air reaching the vial through said duct following inhalation by the patient at an exit duct of the aerosol. As the experts in the sector know, the characteristic parameters of a therapeutic aerosol are the Mass Median Aerodynamic Diameter (MMAD), the Geometric Standard Deviation (GSD), and the rate or speed of nebulisation.

The MMAD provides an indication of the mean dimensions of the particles forming the aerosol, this making it possible to forecast which part of the patient's airways the nebulised medication will deposit in.

The GSD permits an assessment of the degree of dispersion of the dimensions of the particles of solution within the distribution. Lastly, the rate of nebulisation is substantially an index of the mass of nebulised medication in the unit of time.

The MMAD and GSD can both be calculated from the distribution of the diameters of the particles of aerosol: the MMAD is merely the aerodynamic diameter to which 50% of the distribution of the diameters of the particles of the aerosol solution correspond; the GSD can be calculated from the graph of the distribution of the diameters of the particles, if the distribution is sufficiently rectilinear between 10% and 90%, that is to say if the distribution is Gaussian, using suitable extrapolation calculation methods (see ISO 9276-2).

Medical literature has established that for therapeutic purposes the regions of the respiratory tree of a patient which can be reached by the aerosol are related to the dimensions of the medical particles inhaled. More specifically, aerodynamic diameters of the particles of over 5 micrometres are suitable for treating the upper airways; diameters of 2 to 6 micrometres for the tracheo-bronchial region; diameters of 0.5 to 3 micrometres for alveolar administration.

From the above findings it is evident that to treat respiratory disorders it is important that the dose of medication is administered only in the region of the respiratory tree of therapeutic interest, so as to avoid wasting medication, as well as to prevent undesired systemic effects.

To achieve this, a nebulizer vial able to generate an aerosol with specific "particle size" characteristics must be used. The need is therefore deeply felt to make a nebulizer vial for aerosol therapy making it possible to vary the MMAD.

It is also important for the nebulizer vial to have a nebulisation rate suitable for the patient undergoing treatment. In fact, while true that increasing the nebulisation rate makes treatment faster, a high rate of nebulisation, while being suitable for normal adult patients, may be excessive for specific categories of patient, such as children or serious asthma sufferers, so much so as to make it difficult to correctly administer the medication.

It is therefore desirable to make a nebulizer vial of the type specified above in which the rate of nebulisation may be varied.

Since a nebulizer vial for aerosol therapy is often used for treatment at home by inexpert persons and may be used at different times for different respiratory disorders, one particular need is to make a vial of the aforesaid type in which the variation of the characteristics of the aerosol (particle size and nebulisation rate) depending on the therapeutic needs of the patient can be achieved very simply, so as to be performed by any patient.

In a previous patent application, no. EP1307255A1 of the same Applicant, in order to satisfy the aforesaid requirement a nebulizer vial was proposed in which, for the same diameter of the orifice of the nozzle, means of varying the distance between the orifice of the nozzle and the flow-breaker device are provided for. It has in fact been verified that the variation of this distance makes the MMAD of the particles of solution vary.

In particular, according to said patent application, one way of varying the MMAD of the particles of the aerosol produced by the vial consists of providing nozzles of different lengths which can be substituted, so that, depending on the length of the nozzle, the distance between the orifice of the nozzle and the flow-breaker diaphragm varies. Another way of varying the aforesaid distance consists of making it possible to insert the tube of compressed air further in or not in the reservoir of the medication solution, so that the orifice of the nozzle (which in this case is fixed in relation to the tube) is closer or further to/from the flow-breaker diaphragm. Another way of regulating the aforesaid distance consists of making a casing of the vial in two parts, which can insert into each other or screw to each other so as to vary the reciprocal distance.

As a further variant, several spacer connection trunks of varying height may be provided between the two parts of the vial body.

Also, the external air channel (at the bottom end of which the flow-breaker diaphragm is attached) may be made so as to be vertically movable in relation to the rest of the vial, between specific limits, so as to vary the aforesaid distance.

Such technical solutions are not without drawbacks however. In particular, the use of different interchangeable nozzles or different spacer trunks has the disadvantage of increasing the number of components of the appliance, among other things increasing the risk of losing or damaging one or other of such components. The technical solutions described in any case require a more complex vial structure composed of a large number of parts. For example, the embodiment providing for inserting the tube of compressed air further into the reservoir of the medication solution or not does not make it possible to produce such components in one piece which would instead be desirable so as to simplify the production, assembly and maintenance of the appliance.

The object of the present invention is to propose a vial for aerosol therapy which is able to satisfy the aforementioned functional requirements and at the same time which is free of the limitations of the prior technical solutions of vial currently known.

Such object is achieved by a nebulizer vial for aerosol therapy according to claim 1. The dependent claims describe preferred or advantageous embodiments of the vial according to the invention.

Further characteristics and advantages of the vial according to the invention will in any case be evident from the description given below of its preferred and non-limiting embodiments, with reference to the appended drawings, wherein:
- Figure 1 is an exploded perspective view of the nebulizer vial according to the invention;
- Figure 2 is a perspective view of the assembled vial, with the multi-nozzle unit in the lowered position of use;

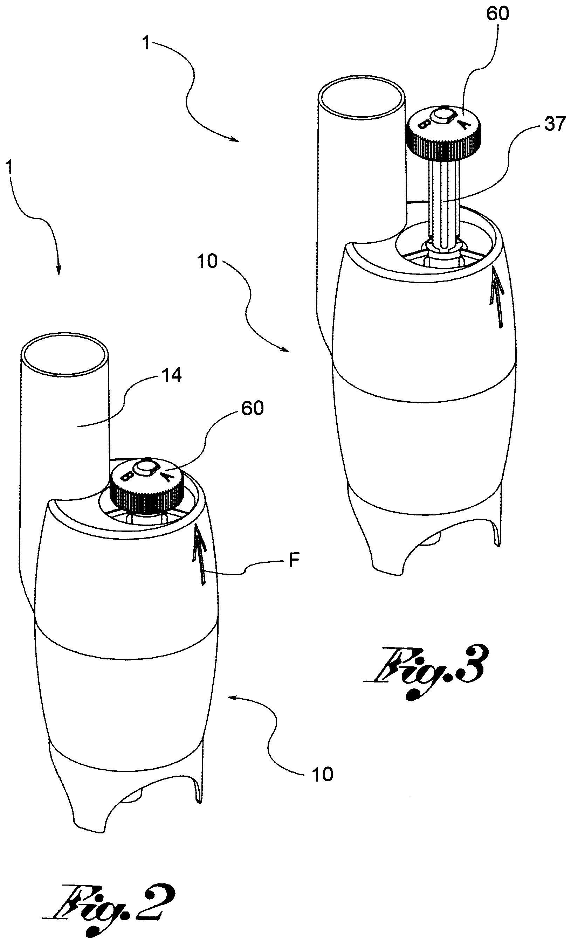

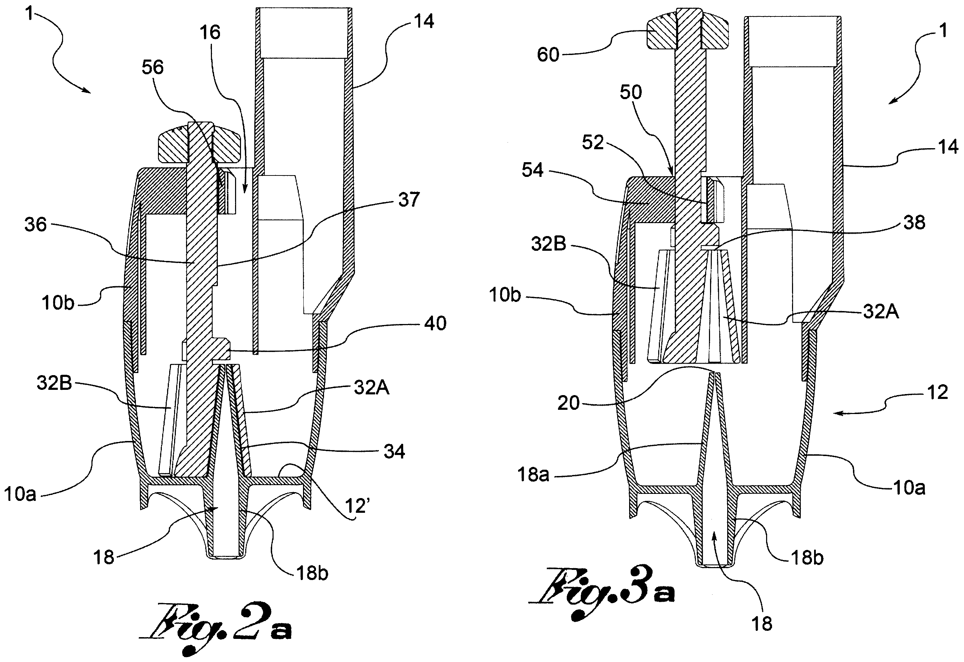

- Figure 2 is an axial cross-section of the vial in figure 2;
- Figure 3 is a perspective view of the assembled vial, with the multi-nozzle unit in the raised switchover position; and
- Figure 3a is an axial cross-section of the vial in figure 3.

In said drawings, reference numeral 1 globally denotes a nebulizer vial for aerosol therapy according to the invention.

The vial 1 comprises a vial body 10 defining a reservoir 12 suitable for containing a medication solution for aerosol therapy. Considering the vial oriented vertically, that is in the position of normal use, in a preferred embodiment, the vial body 10 comprises a lower cup-shaped part 10a, defining the reservoir 12 for the medication solution, and an upper part 10b, placed so as to close said lower part 10a. The two lower 10a and upper 10b parts are provided with reciprocal connections means, for example of the threaded, bayonet, snap, pressure type etc.

The vial body 10 defines an exit duct 14 of the aerosol, suitable for connecting for example to a mask - not shown- and an inlet aperture 16 of air coming from outside. For example, the exit duct 14 of the aerosol and the inlet aperture 16 of the air are made in the upper part 10b of the vial body 10.

The reservoir 12 for the medication solution is crossed in a sealed manner by a tube 18 for the supply of compressed air generated by a compressor (not shown) of the type conventionally used for aerosol appliances. In particular, the tube 18 has a distal or upper portion 18a, which extends into the reservoir 12, emerging for example from a lower wall 12' of the vial body which forms the bottom of said reservoir 12, and a proximal or lower end 18b, suitable for connecting to the compressor.

The distal portion 18a of the tube is tapered internally so as to form an exit orifice 20 suitable to cause a Venturi effect. In addition, the distal portion 18a of the tube is also tapered externally. As explained above, the external tapering facilitates the formation, when a nozzle (or "pisper") is concentrically fitted on said distal portion which defines with the outer wall of said distal portion of the tube an interspace communicating at the bottom with the solution contained in the reservoir, of a flow of particles of solution directed upwards, which comes out of the orifice of the nozzle.

According to the invention, the vial comprises a multi-nozzle group 30 associable to the distal portion 18a of the supply tube 18 of the compressed air. Such multi-nozzle unit 30 comprises at least two nozzles 32A, 32B, 32C, each suitable to be concentrically fitted onto said distal portion 18a of the tube 18so that an interspace 34 remains between the tube and nozzle in fluidic communication with the medication solution. Said nozzles 32A-32C are differently sized and/or configured so as to produce a different atomisation of the medication solution. In addition, said nozzles are integral with each other and with control means suitable for permitting a movement of said nozzles inside the vial body 10 for their selective coupling to the distal portion 18a of the tube 18.

In other words, one aspect of the invention is to permit the varying of the MMAD of the particles forming the aerosol using at least two different types of nozzle, which however form part of a single device easy for the user to manoeuvre to select the desired nozzle to fit onto the distal portion of the tube.

In one embodiment, the nozzles 32A-32C are alongside each other, preferably coplanar in relation to a horizontal plane.

In a preferred embodiment, the nozzles 32A-32C are joined to a common control stem 36. For example, said nozzles are attached to a lower portion of said control stem 36. Preferably, the nozzles are angularly equidistant from the axis of said control stem 36. In the example shown, the multi-nozzle unit 30 is fitted with three nozzles 32A-32C.

In a preferred embodiment, the control stem 36 with the nozzles joined to it is translatable along a longitudinal axis parallel to the tube axis 18 between a lowered position of use, in which the nozzles 32A-32C are selectively fitted onto the distal portion of tube 18a, and a raised switchover position, in which said nozzles 32A-32C are disengaged from said distal portion of tube 18a. When the multi-nozzle unit 30 is in the raised switchover position, the control stem 36 is rotatable around said longitudinal axis so as to selectively position the nozzles coaxially to the tube axis.

In a preferred embodiment, each nozzle 32A-32C ends above with a nozzle orifice 38 above which a flow-breaker device 40 is positioned. According to one aspect of the invention, each flow-breaker device 40 is integral with the multi-nozzle unit 30. For example, each flow-breaker device 40 comprises a crossbar which extends radially from the control stem 36. In one embodiment, a different atomisation of the particles which form the aerosol is obtained by varying the distance between the flow breaker devices 40 and the orifices 38 of the nozzles. As a result, the flow-breaker devices 40 are positioned at different distances from the respective nozzle orifices 38 in the multi-nozzle unit 30.

Within the scope of the present invention therefore, the term "nozzle" is understood to define not only as the truncated-cone element surrounding the distal portion of tube 18a and ending with the orifice 38, but also the flow-breaker device 40 positioned above the orifice 38 of the nozzle. Consequently, when specifying that the nozzles are differently sized and/or configured so as to produce a different atomisation of the medication solution, the case in which the truncated-cone elements surrounding the distal portion of tube are identical to each other and only the distance from the relative flow-breaker devices 40 changes is also understood as included.

According to another aspect of the invention, the multi-nozzle unit 30 and the vial body 10 are provided with reciprocal coupling means suitable to establish the plurality of angular positions of said multi-nozzle unit, one for each nozzle.

For example, in one embodiment, an annular guide element 50 is placed in the inlet aperture 16 of the air. Said annular guide element 50 has an axial hole 52 and is connected to the vial body 10 for example by means of radial arms 54. The control stem 36 is inserted in said axial hole 52 with the possibility of axial translation. A portion of said stem 36 is fitted with longitudinal ribs 37 suitable to engage corresponding radial recesses 56 made in said annular guide element 50 when the multi-nozzle element is in a lowered position (Figures 2 and 2a). When instead the multi-nozzle unit 30 is in the raised switchover position, the longitudinal ribs 37 disengage from the respective radial recesses 56 and the control stem 36 can be rotated (Figures 3 and 3a).

It is to be noted, as shown in Figure 3a, that the flow-breaker devices 40 in the position of maximum raising of the multi-nozzle unit abut against the annular guide element 50 and thus also act as stop means to the axial translation of the control stem 36.

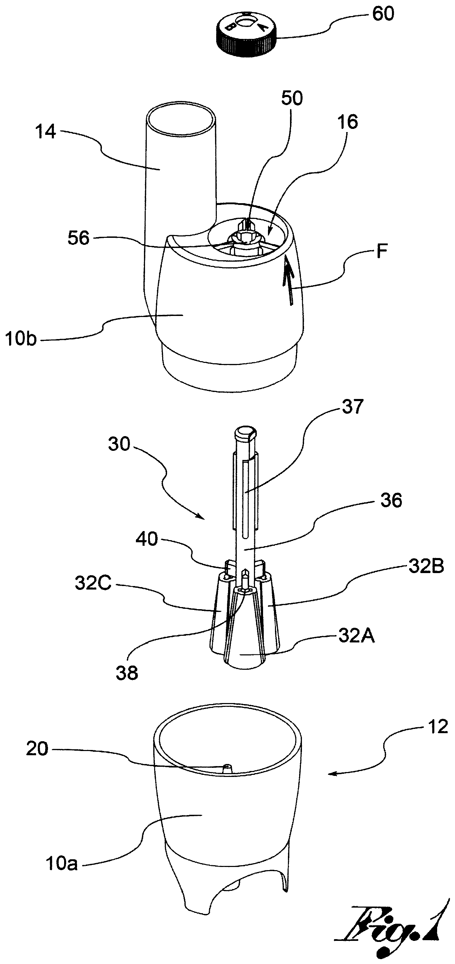

In a preferred embodiment, the distal end of the command stem 36 projects from the air inlet aperture 16 so as to be accessible from outside for manual movement of the multi-nozzle unit 30. In particular, said distal end of the control stem 36 is fitted with a gripping member 60 indicating the radial positions of the nozzles 32A-32C. Preferably, a reference mark made in the vial body 10, for example shown by the arrow F in Figures 1-3, indicates which nozzle is threaded onto the distal portion of tube 18a.

The functioning of the vial according to the invention clearly derives from what has been said above.

Depending on the type of therapy to be administered, the patient will know which nozzle is to be used. If the nozzle already coupled to the distal portion of tube is not the right one, the patient needs only to raise the multi-nozzle unit, for example using the gripping member joined to the control stem, to the limit stop position. At this point, the control stem may be rotated so as to align the indication of the desired nozzle on the gripping member with the reference mark on the vial body. This angular alignment operation is facilitated by the presence of the longitudinal ribs of the control stem which interact with the respective radial recesses of the annular guide element.

This way, the correct nozzle comes to find itself coaxial with the axis of the compressed air tube. The multi-nozzle unit can then be lowered for use.

With a single device, the multi-nozzle unit, the patient thus has available a plurality of nozzles to vary the atomisation of the particles of the aerosol. Selecting the right nozzle is simple and does not require the separation of such unit from the vial body.

Advantageously, the multi-nozzle unit is made in one piece, for example from plastic, by moulding.

Advantageously, in addition, the compressed air supply tube is made in one piece with the reservoir of medication solution. The appliance for aerosol therapy thus proves composed of a reduced number of components, has a simple structure and is therefore reliable as well as easy to clean and maintain.

A person skilled in the art may make modifications and adaptations to the nebulizer vial according to the invention so as to satisfy contingent requirements, replacing elements with others functionally equivalent, while remaining within the scope of protection of the following claims. Each of the characteristics described as belonging to a possible embodiment may be realised independently of the other embodiments described.

## Claims

1. Nebulizer vial for aerosol therapy, comprising:
- a vial body (10) defining a reservoir (12) suitable for containing a medication solution for aerosol therapy, an exit duct (14) of the aerosol, and an inlet aperture (16) of air coming from outside;
- a tube (18) having a distal portion (18a) which extends into said reservoir and a proximal end (18b) suitable for connecting to a compressed air source, said distal portion (18a) tapering so as to form an exit orifice (20) suitable to generate a Venturi effect,
- a multi-nozzle unit (30) comprising at least two nozzles (32A, 32B, 32C), each suitable to be concentrically fitted onto said distal portion (18a) of the tube so that an interspace remains between the tube and nozzle in fluidic communication with the medication solution, said nozzles (32A, 32B, 32C) being differently sized and/or configured so as to produce a different atomisation of the medication solution,
**characterised in that** said nozzles (32A, 32B,...) are integral with each other and with control means suitable for permitting a movement of said nozzles inside the vial body (10) for their selective coupling to the distal portion (18a) of tube (18).

2. Vial according to claim 1, wherein said nozzles (32A, 32B, 32C) are alongside each other and integral with a common control stem (36), said stem being translatable along a longitudinal axis parallel to the tube axis between a lowered position of use, in which the nozzles (32A, 32B, 32C) are selectively fitted onto said distal portion (18a) of tube (18), and a raised switchover position, in which said nozzles (32A, 32B, 32C) are disengaged from said distal portion of tube, said stem (36) being rotatable around said longitudinal axis so as to selectively position said nozzles coaxially to the tube axis.

3. Vial according to claim 1 or 2, wherein each nozzle (32A, 32B, 32C) ends above with a nozzle orifice (38) above which a flow-breaker device (40) is positioned.

4. Vial according to the previous claim, wherein each flow-breaker device (40) is integral with the multi-nozzle unit (30).

5. Vial according to claims 2 and 4, wherein each flow-breaker device (40) comprises a crossbar which extends radially from the control stem (36).

6. Vial according to any of the claims 3-5, wherein the flow-breaker devices (40) are positioned at different distances from the respective nozzle orifices (38).

7. Vial according to claim 2 or any of claims 3-6 when dependent on claim 2, wherein the multi-nozzle unit (30) and the vial body (10) are provided with reciprocal coupling means suitable to establish a plurality of angular positions of said multi-nozzle unit (30), one of said angular positions for each nozzle (32A, 32B, 32C).

8. Vial according to the previous claim, wherein said control stem (36) is inserted in an axial hole (52) of an annular guide element (50) connected to the vial body (10) and extending into the air inlet aperture (16), a portion of said stem (36) being fitted with longitudinal ribs (37) suitable to engage corresponding radial recesses (56) made in said annular guide element (50) when the multi-nozzle unit (30) is in a lowered position.

9. Vial according to the previous claim, wherein the distal end of the control stem (36) projects from the air inlet aperture (16) so as to be accessible from outside for manual movement of the multi-nozzle unit (30).

10. Vial according to the previous claim, wherein said distal end of the control stem (36) is fitted with a gripping member (60) indicating the radial positions of the nozzles (32A, 32B, 32C).

11. Vial according to any of the previous claims, wherein the multi-nozzle unit (30) is made in one piece in a single body.

12. Vial according to any of the previous claims, wherein the tube (18) is made in one piece with the reservoir (12) of medication solution.

## Patentansprüche

1. Vernebelungsphiole für die Aerosoltherapie, die umfasst:
- einen Phiolenkörper (10), der einen Vorratsbehälter (12), der geeignet ist, um eine Medikamentenbehandlungslösung für die Aerosoltherapie zu enthalten, einen Ausgangskanal (14) für das Aerosol und eine Einlassöffnung (16) für von außen kommende Luft definiert;
- ein Rohr (18) mit einem distalen Abschnitt (18a), der sich in den Vorratsbehälter erstreckt, und einem proximalen Ende (18b), das zum Verbinden mit einer Druckluftquelle geeignet ist, wobei der distale Abschnitt (18a) angeschrägt ist, um eine Austrittsmündung (20) zu bilden, die geeignet ist, um einen Venturi-Effekt zu erzeugen,
- eine Mehrdüseneinheit (30), die wenigstens zwei Düsen (32A, 32B, 32C) umfasst, von denen jede geeignet ist, konzentrisch auf den distalen Abschnitt (18A) des Rohrs montiert zu werden, so dass ein Zwischenraum zwischen dem Rohr und der Düse in einer Fluidverbindung mit der Medikamentenbehandlungslösung verbleibt, wobei die Düsen (32A, 32B, 32C) unterschiedlich dimensioniert und/oder aufgebaut sind, um eine unterschiedliche Zerstäubung der Medikamentenlösung zu erzeugen,
**dadurch gekennzeichnet, dass** die Düsen (32A, 32B, ...) integral miteinander und mit einem Steuermittel sind, das geeignet ist, für ihre selektive Kopplung mit dem distalen Abschnitt (18a) des Rohrs (18) eine Bewegung der Düsen im Inneren des Phiolenkörpers (10) zuzulassen.

2. Phiole nach Anspruch 1, wobei die Düsen (32A, 32B, 32C) längsseits voneinander und integral mit einem gemeinsamen Steuerschaft (36) sind, wobei der Schaft entlang einer Längsachse parallel zu der Rohrachse zwischen einer abgesenkten Verwendungsposition, in der die Düsen (32A, 32B, 32C) selektiv auf den distalen Abschnitt (18a) des Rohrs (18) montiert sind, und einer erhöhten Umschaltposition, in der die Düsen (32A, 32B, 32C) von dem distalen Abschnitt des Rohrs gelöst sind, beweglich ist, wobei der Schaft (36) um die Längsachse drehbar ist, um die Düsen selektiv koaxial zu der Rohrachse zu positionieren.

3. Phiole nach Anspruch 1 oder 2, wobei jede Düse (32A, 32B, 32C) oberhalb mit einer Düsenmündung (38) endet, oberhalb welcher eine Strömungsunterbrechervorrichtung (40) positioniert ist.

4. Phiole nach dem vorhergehenden Anspruch, wobei jede Strömungsunterbrechervorrichtung (40) integral mit der Mehrdüseneinheit (30) ist.

5. Phiole nach den Ansprüchen 2 und 4, wobei jede Strömungsunterbrechervorrichtung (40) eine Querstange umfasst, die sich von dem Steuerschaft (36) radial erstreckt.

6. Phiole nach einem der Ansprüche 3 - 5, wobei die Strömungsunterbrechervorrichtungen (40) in unterschiedlichen Abständen von den jeweiligen Düsenmündungen (38) positioniert sind.

7. Phiole nach Anspruch 2 oder einem der Ansprüche 3 bis 6, in Abhängigkeit von Anspruch 2, wobei die Mehrdüseneinheit (30) und der Phiolenkörper (10) mit gegenseitigen Kopplungsmitteln versehen sind, die geeignet sind, eine Vielzahl von Winkelpositionen der Mehrdüseneinheit (30), eine der Winkelpositionen für jede Düse (32A, 32B, 32C), einzurichten.

8. Phiole nach dem vorhergehenden Anspruch, wobei der Steuerschaft (36) in ein Axialloch (52) eines ringförmigen Führungselements (50) eingesetzt ist, das mit dem Phiolenkörper (10) verbunden ist und sich in die Lufteinlassöffnung (16) erstreckt, wobei ein Abschnitt des Schafts (36) mit Längsrippen (37) versehen ist, die geeignet sind, in entsprechende radiale Vertiefungen (56), die in dem ringförmigen Führungselement (50) hergestellt sind, einzugreifen, wenn die Mehrdüseneinheit (30) in einer gesenkten Position ist.

9. Phiole nach dem vorhergehenden Anspruch, wobei das distale Ende des Steuerschafts (36) von der Lufteinlassöffnung (116) vorsteht, um für die manuelle Bewegung der Mehrdüseneinheit (30) von außen zugänglich zu sein.

10. Phiole nach dem vorhergehenden Anspruch, wobei das distale Ende des Steuerschafts (36) mit einem Greifelement (60) versehen ist, das die radialen Positionen der Düsen (32A, 32B, 32C) anzeigt.

11. Phiole nach einem der vorhergehenden Ansprüche, wobei die Mehrdüseneinheit (30) in einem Stück in einem einzigen Körper hergestellt ist.

12. Phiole nach einem der vorhergehenden Ansprüche, wobei das Rohr (18) in einem Stück mit dem Vorratsbehälter (12) der Medikamentenbehandlungslösung hergestellt ist.

## Revendications

1. Flacon nébuliseur pour thérapie par aérosol, comprenant :
- un corps de flacon (10) définissant un réservoir (12) approprié à contenir une solution de médicament pour une thérapie par aérosol, un conduit de sortie (14) de l'aérosol, et une ouverture d'entrée (16) d'air provenant de l'extérieur ;
- un tube (18) ayant une partie distale (18a) qui s'étend dans ledit réservoir et une extrémité proximale (18b) appropriée à se raccorder à une source d'air comprimé, ladite partie distale (18a) se rétrécissant progressivement pour former un orifice de sortie (20) approprié à générer un effet Venturi,
- une unité à buses multiples (30) comprenant au moins deux buses (32A, 32B, 32C), chacune appropriée à être ajustée concentriquement sur ladite partie distale (18a) du tube de telle sorte qu'un espace reste entre le tube et la buse en communication fluidique avec la solution de médicament,
lesdites buses (32A, 32B, 32C) étant dimensionnées et/ou conçues différemment pour produire une atomisation différente de la solution de médicament,
**caractérisé en ce que** lesdites buses (32A, 32B, ...) sont formées en un seul tenant les unes avec les autres et avec un moyen de commande approprié pour permettre un mouvement desdites buses à l'intérieur du corps de flacon (10) pour leur couplage sélectif à la partie distale (18a) du tube (18).

2. Flacon selon la revendication 1, dans lequel lesdites buses (32A, 32B, 32C) sont les unes le long des autres et en un seul tenant avec une tige de commande commune (36), ladite tige pouvant être translatée le long d'un axe longitudinal parallèle à l'axe de tube entre une position d'utilisation abaissée, dans laquelle les buses (32A, 32B, 32C) sont sélectivement ajustées sur ladite partie distale (18a) du tube (18), et une position de permutation surélevée, dans laquelle lesdites buses (32A, 32B, 32C) sont désolidarisées de ladite partie distale du tube, ladite tige (36) pouvant être entraînée en rotation autour dudit axe longitudinal pour sélectivement positionner lesdites buses coaxialement à l'axe du tube.

3. Flacon selon la revendication 1 ou 2, dans lequel chaque buse (32A, 32B, 32C) se termine au-dessus avec un orifice de buse (38) au-dessus duquel est positionné un dispositif d'interruption de flux (40).

4. Flacon selon la revendication précédente, dans lequel chaque dispositif d'interruption de flux (40) est formé en un seul tenant avec l'unité à buses multiples (30).

5. Flacon selon les revendications 2 et 4, dans lequel chaque dispositif d'interruption de flux (40) comprend une barre transversale qui s'étend radialement à partir de la tige de commande (36).

6. Flacon selon l'une quelconque des revendications 3 à 5, dans lequel les dispositifs d'interruption de flux (40) sont positionnés à différentes distances des orifices de buses respectifs (38).

7. Flacon selon la revendication 2 ou l'une quelconque des revendications 3 à 6 lorsqu'elles dépendent de la revendication 2, dans lequel l'unité à buses multiples (30) et le corps de flacon (10) sont dotés d'un moyen de couplage réciproque approprié à établir une pluralité de positions angulaires de ladite unité à buses multiples (30), une desdites positions angulaires pour chaque buse (32A, 32B, 32C).

8. Flacon selon la revendication précédente, dans lequel ladite tige de commande (36) est insérée dans un trou axial (52) d'un élément de guidage annulaire (50) raccordé au corps de flacon (10) et s'étendant dans l'ouverture d'entrée d'air (16), une partie de ladite tige (36) étant dotée de nervures longitudinales (37) appropriées à se mettre en prise avec des creux radiaux correspondants (56) formés dans ledit élément de guidage annulaire (50) lorsque l'unité à buses multiples (30) se trouve dans une position abaissée.

9. Flacon selon la revendication précédente, dans lequel l'extrémité distale de la tige de commande (36) se projette de l'ouverture d'entrée d'air (16) pour être accessible depuis l'extérieur pour le mouvement manuel de ladite unité à buses multiples (30).

10. Flacon selon la revendication précédente, dans lequel ladite extrémité distale de la tige de commande (36) est dotée d'un élément de préhension (60) indiquant les positions radiales des buses (32A, 32B, 32C).

11. Flacon selon l'une quelconque des revendications précédentes, dans lequel l'unité à buses multiples (30) est formée en un seul tenant en un seul corps.

12. Flacon selon l'une quelconque des revendications précédentes, dans lequel le tube (18) est formé en un seul tenant avec le réservoir (12) de solution de médicament.
